# FASCICULE DE BREVET EUROPEEN

(11) **EP 0 774 250 B1**
(45) Date de publication et mention de la délivrance du brevet: **25.11.1998**
(21) Numéro de dépôt: 96402291.7
(22) Date de dépôt: 28.10.1996
(51) Int. Cl.: A61K 31/00, A61K 31/44, A61K 31/52, A61K 39/395

(54) **Utilisation d'un antagoniste de TNF-alpha pour le traitement des rougeurs cutanées d'origine neurogène**
Verwendung eines Antagonisten von TNF-alpha zur Behandlung von Hautrötungen neurogenen Ursprungs
Use of a TNF alpha antagonist against skin redness of neurogenic origin

(30) Priorité: 20.11.1995 FR 9513729
(43) Date de publication de la demande: 21.05.1997
(73) Titulaire: L'OREAL, 75008 Paris (FR)
(72) Inventeur: De Lacharriere, Olivier, 75015 Paris (FR); Breton, Lionel, 78000 Versailles (FR)
(74) Mandataire: Lhoste, Catherine

(56) Documents cités:
- EP-A- 0 680 749
- J. INVEST. DERMATOL., vol. 102, no. 6, 1994, pages 934-937, XP000576630 G. SENALDI ET AL.: "Protective effect of N-acetylcysteine in hapten-induced irritant and contact hypersensitivity reactions."
- J. EXP. MED., vol. 173, no. 3, 1991, pages 673-679, XP000576859 P.F. PIGUET ET AL.: "Tumor necrosis factor is a critical mediator in hapte-induced irritant and contact hypersensitivity reactions."
- AGENTS & ACTIONS, vol. 38, no. s.i. II, 1993, pages C212-C214, XP000576409 N.A. HAYES ET AL.: "The action of a calcitonin gene-related peptide antagonist in human skin."
- SEMIN. DERMATOL., vol. 10, no. 3, 1991, pages 138-147, XP000576399 J.T. APGAR: "Newer aspects of inflammatory bowel disease and its cutaneous manifestations: a selective review."
- CELL. IMMUNOL., vol. 161, no. 2, 1995, pages 288-294, XP000576633 N.HIGASHI ET AL.: "Involvement of inflammatory cytokines in a delayed-type hypersensitivity reaction."

## Description

La présente invention se rapporte à l'utilisation d'un antagoniste de TNF-alpha (Tumor Necrosis Factor alpha en langue anglosaxonne) dans ou pour la préparation d'une composition cosmétique, pharmaceutique et/ou dermatologique à application topique, pour traiter les rougeurs de la peau d'origine neurogène et particulièrement la rosacée.

La rosacée est une affection cutanée caractérisée par un érythème du visage prédominant sur les pommettes, le front, le nez, une hyperséborrhée du visage au niveau du front, du nez et des joues, et par une composante infectieuse avec des pustules.

Par ailleurs, il s'associe à ces signes une composante neurogène, c'est-à-dire une hyper-réactivité de la peau du visage et du cou, caractérisée par l'apparition de rougeurs et de sensations subjectives du type démangeaisons ou prurit, de sensations de brûlures ou d'échauffement, de sensations de picotements, de fourmillements, d'inconforts, de tiraillements, etc.

Ces signes d'hyper-réactivité peuvent être déclenchés par des facteurs très divers tels que la prise d'aliments, de boissons chaudes ou alcoolisées, par des variations de température rapide, par la chaleur et notamment l'exposition aux ultraviolets ou aux infrarouges, par une humidité relative basse, par l'exposition de la peau aux vents violents ou aux courants d'air (soufflerie, air conditionné), par l'application de tensioactifs ou autres composés même lorsque ceux-ci ne sont pas connus comme particulièrement irritants.

Ces rougeurs qui peuvent persister plusieurs heures après l'exposition de la peau au stimulus, sont souvent disgracieuses et créent souvent une gêne psychologique notable.

Jusqu'alors, le mécanisme de déclenchement de ces signes était très mal connu et la rosacée était traitée par des actifs tels que les antiséborrhéiques et les anti-infectieux, par exemple le peroxyde de benzoyle, l'acide rétinoïque, le métronidazol ou les cyclines, qui agissaient sur l'infection et l'hyperséborrhée, mais ne permettraient pas de traiter la composante érythémateuse de cette affection ainsi que l'hyper-réactivité de la peau.

Il subsiste donc le besoin d'un traitement de la rougeur cutanée, composante essentielle de l'état d'hyper-réactivité de la peau atteinte de rosacée.

La demanderesse a trouvé que la rougeur cutanée pouvait être traitée par application topique d'antagonistes de TNF-alpha.

L'article dans le J. INVESTIV. DERMATOL., vol. 102,no.6, 1994 décrit l'effet protecteur de la N-acétylcystéïne dans l'irritation induite par un haptène et dans les réactions d'hypersensibilité de contact.

Personne n'avait envisagé jusqu'à ce jour d'utiliser des antagonistes de TNF-alpha pour traiter la rougeur cutanée, notamment la rosacée.

Aussi, la présente invention a pour objet l'utilisation d'au moins un antagoniste de TNF-alpha dans ou pour la préparation d'une composition cosmétique, pharmaceutique ou dermatologique à application topique, pour traiter les rougeurs cutanées d'origine neurogène.

La présente invention a plus particulièrement pour objet l'utilisation d'un antagoniste de TNF-alpha dans ou pour la préparation d'une composition cosmétique, pharmaceutique ou dermatologique à application topique, pour traiter la rosacée.

L'application de compositions contenant un ou des antagonistes de TNF-alpha permet d'obtenir une nette diminution voire une disparition complète de la rougeur qui se manifeste notamment dans la rosacée.

La présente invention a aussi pour objet un procédé de traitement cosmétique, dermatologique ou pharmaceutique des rougeurs cutanées d'origine neurogène, caractérisé par le fait que l'on applique sur la peau, sur le cuir chevelu et/ou sur les muqueuses, une composition contenant au moins un antagoniste de TNF-alpha dans un milieu cosmétiquement, dermatologiquement ou pharmaceutiquement acceptable.

La présente invention a encore pour objet un procédé de traitement cosmétique, dermatologique ou pharmaceutique de la rosacée, caractérisé par le fait que l'on applique sur la peau, sur le cuir chevelu et/ou sur les muqueuses, une composition contenant au moins un antagoniste de TNF-alpha dans un milieu cosmétiquement, dermatologiquement ou pharmaceutiquement acceptable.

La composition de l'invention contient un milieu cosmétiquement, pharmaceutiquement ou dermatologiquement acceptable, c'est-à-dire un milieu compatible avec la peau, les ongles, les muqueuses, les tissus et les cheveux. La composition contenant l'antagoniste de TNF-alpha peut être appliquée par voie topique sur le visage, le cou, les cheveux, les muqueuses et les ongles, les grands plis ou toute autre zone cutanée du corps.

On entend par antagoniste de TNF-alpha, toute substance susceptible d'inhiber la libération et/ou la synthèse et/ou la fixation réceptorielle de TNF-alpha.

Selon l'invention, il est possible d'utiliser un seul ou conjointement plusieurs antagonistes du TNF-alpha.

Pour qu'une substance soit reconnue comme un antagoniste réceptoriel de TNF-alpha, elle doit répondre notamment aux caractéristiques suivantes :
- avoir une affinité sélective pour les récepteurs spécifiques du TNF-alpha ;
- avoir une activité pharmacologique antagoniste réceptoriel de TNF-alpha, c'est-à-dire induire une réponse pharmacologique cohérente dans les tests suivants : inhibition de l'adhésion de macrophages induite par TNF-alpha sur les cellules endothéliales ou inhibition de la libération d'anions superoxydes induite par TNF-alpha sur les neutrophiles ou inhibition de l'activité mitogène du TNF-alpha sur les fibroblastes du derme.

Pour qu'une substance soit reconnue comme un antagoniste de la libération et/ou de la synthèse de TNF-alpha, elle doit répondre notamment à la caractéristique suivante : >
- inhibition de la libération de TNF-alpha par des monocytes (cellules U937) différenciés par un ester de phorbol (PMA).

Les antagonistes de TNF-alpha peuvent être des molécules de synthèse organiques ou des molécules minérales ou des extraits de produits naturels (végétaux ou animaux).

Les antagonistes réceptoriels de TNF-alpha et les inhibiteurs de la libération et/ou de la synthèse de TNF-alpha utilisables dans l'invention sont en particulier la lisophylline, l'A802715, la sulfasalazine, le CDP-571 (anticorps anti-TNF-alpha), le MDL-201112.

Dans les compositions selon l'invention, les antagonistes de TNF-alpha sont utilisés de préférence en une quantité allant de 0,000001 à 10 % en poids par rapport au poids total de la composition, et en particulier en une quantité allant de 0,0001 à 5 % en poids par rapport au poids total de la composition.

De façon avantageuse, on associe aux antagonistes de TNF-alpha un ou plusieurs antagonistes d'un ou plusieurs neuropeptides et/ou un ou plusieurs antagonistes d'un ou plusieurs médiateurs de l'inflammation.

Comme antagonistes de neuropeptides utilisables dans l'invention, on peut citer les antagonistes de substance P et les antagonistes de CGRP. De façon avantageuse, on utilise des antagonistes réceptoriels de substance P et/ou de CGRP.

La substance P est un élément chimique polypeptidique élaboré et libéré par une terminaison nerveuse. Elle fait partie de la famille des tachykinines qui proviennent des terminaisons nerveuses libres de l'épiderme et du derme. La substance P intervient notamment dans la transmission de la douleur et dans des maladies du système nerveux central telles que l'anxiété, la schizophrénie, dans des maladies respiratoires et inflammatoires, dans des maladies gastro-intestinales, dans des maladies rhumatismales et dans certaines maladies dermatologiques telles que l'eczéma, le psoriasis, l'urticaire et les dermites de contact.

Comme antagoniste de substance P utilisable dans l'invention, on peut citer toute substance d'origine organique ou minérale, capable de produire une inhibition de la fixation réceptorielle de substance P ou une inhibition de la synthèse et/ou la libération de substance P par des fibres nerveuses sensitives.

Pour qu'une substance soit reconnue comme un antagoniste de substance P, elle doit répondre notamment à la caractéristique suivante :
- avoir une activité pharmacologique antagoniste de la substance P, c'est-à-dire induire une réponse pharmacologique cohérente notamment dans l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste réceptoriel de substance P peut être un peptide ou un dérivé non peptidique comportant un hétéroatome. ll peut être choisi aussi parmi les sels et parmi les extraits d'origine végétale et/ou bactérienne.

On peut utiliser par exemple comme peptide antagoniste réceptoriel de substance P le sendide et le spantide II.

On peut également utiliser dans l'invention comme peptide ceux décrits dans les documents US-A-4472305, US-A4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Comme dérivé non peptidique comportant un hétéroatome, on peut utiliser par exemple des composés hétérocycliques notamment soufrés, azotés ou oxygénés, des composés comprenant un atome d'azote lié directement ou indirectement à un cycle benzénique.

Comme composé hétérocyclique, on peut citer par exemple ceux comportant un hétérocycle azoté, décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151 , WO-A-92/15585, WO-A-92/17449, WO-A-92/20676, WO-A-93/00330, WO-A-93/00331 , WO-A-93/01159, WO-A-93/01169, W0-A-93/01170, WO-A-93/06099, WO-A-93/09116, WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolylbenzofuranne-carboxamides ou les alcoxy- et/ou aryloxy- tétrazolylbenzothiophène-carboxamides.

Comme composés comportant un atome d'azote lié directement ou indirectement à un noyau benzénique, on peut citer ceux décrits dans les documents suivants : EP-A-522808, WO-A-93/01165 et WO-A-93/10073.

Comme sels, on peut utiliser notamment les sels de strontium ou de lanthanides, et par exemple les chlorures, carbonates, borates, nitrates, acétates, hydroxydes, sulfates, les sels d'acides de fruits et les sels d'acides aminés du strontium ou des lanthanides.

Le CGRP (peptide dérivé du gène de la calcitonine : Calcitonin Gene Related Peptide en langue anglosaxonne) est un élément chimique polypeptidique élaboré et libéré par une terminaison nerveuse.

Comme antagoniste de CGRP utilisable dans l'invention, on peut citer toute substance d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit induire une réponse pharmacologique cohérente notamment dans l'un des tests suivants :
- la substance antagoniste doit diminuer la vasodilatation induite par la capsaïcine et/ou par une stimulation électrique antidromique (appliquée sur un nerf afférent) et/ou
- la substance antagoniste doit provoquer une inhibition de la libération de CGRP par les fibres nerveuses sensitives et/ou
- la substance antagoniste doit provoquer une inhibition de la contraction du muscle lisse du canal déférent induite par le CGRP.

On peut utiliser dans l'invention par exemple comme antagoniste réceptoriel de CGRP, le CGRP 8-37 (séquence des acides aminés 8 à 37 de la partie terminale du CGRP), un anticorps anti-CGRP, un extrait d'origine végétale.

Comme antagonistes de médiateur de l'inflammation utilisables dans l'invention, on peut citer notamment les antagonistes d'histamine, les antagonistes d'interleukine 1, les antagonistes de cyclooxygénase et les antagonistes de lipoxygénase.

A titre d'exemple, les antagonistes de neuropeptide et les antagonistes de médiateur de l'inflammation peuvent être utilisés en une quantité allant de 0,000001 à 10 % en poids par rapport au poids total de la composition et mieux de 0,0001 à 5 %.

L'invention a donc encore pour objet une composition cosmétique, pharmaceutique ou dermatologique, caractérisée par le fait qu'elle contient dans un milieu cosmétiquement, pharmaceutiquement ou dermatologiquement acceptable, au moins un antagoniste de TNF-alpha et au moins un antagoniste de neuropeptide et/ou au moins un antagoniste de médiateur de l'inflammation choisi parmi les antagonistes de substance P, les antagonistes de CGRP, les antagonistes de cyclooxygénase et les antagonistes de lipoxygénase.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles dans les domaines considérés.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de protection, de traitement ou de soin pour le visage, pour les mains, pour les pieds, pour les grands plis anatomiques ou pour le corps, des laits corporels de protection ou de soin, des lotions, gels ou mousses pour le soin de la peau et des muqueuses, comme des lotions de nettoyage ou de désinfection, des compositions pour le bain, des compositions contenant un agent bactéricide.

Les compositions peuvent également consister en des préparations solides constituant des savons ou des pains de nettoyage.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique, pharmaceutique ou dermatologique. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines cosmétique, pharmaceutique et dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'alcool éthylique et l'isopropanol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, le rétinol (vitamine A) et ses dérivés, les acides gras essentiels, les céramides, les huiles essentielles.

On peut, entre autre, associer les antagonistes de TNF-alpha à des agents actifs destinés notamment à la prévention et/ou au traitement des affections cutanées. Parmi ces agents actifs, on peut citer à titre d'exemple : >
- les agents modulant la différenciation et/ou la prolifération et/ou la pigmentation cutanée tels que l'acide rétinoïque et ses isomères, le rétinol et ses esters, le rétinal, les rétinoïdes, notamment ceux décrits dans les documents FR-A-2 570 377, EP-A-199636, EP-A-325540, EP-A-402072, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, les anthralines (dioxyanthranol), les anthranoïdes, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide malique, l'acide salicylique, l'acide citrique et de manière générale les acides de fruits, et l'acide n-octanoyl-5-salicylique ; - les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les antimétabolites ;
- les agents pour lutter contre la chute des cheveux comme le monoxidil ;
- les antiseptiques.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits sur la peau, le cuir chevelu et/ou les muqueuses.

Les exemples suivants illustrent l'invention. Dans ces exemples, les proportions indiquées sont des pourcentages en poids.

### Exemple 1 : Crème pour le visage (émulsion huile dans eau)

| | |
|---|---|
| Lisophylline | 0,5 % |
| Stéarate de glycérol | 2 % |
| Polysorbate 60 (Tween 60 vendu par la société ICI) | 1 % |
| Acide stéarique | 1,4 % |
| Triéthanolamine | 0,7 % |
| Carbomer | 0,4 % |
| Cyclométhicone | 8 % |
| Huile de tournesol | 12 % |
| Antioxydant | 0,05 % |
| Conservateur | 0,3 % |
| Eau | qsp 100 % |

### Exemple 2 : Gel émulsionné (émulsion huile dans eau)

| | |
|---|---|
| Cyclométhicone | 3 % |
| Huile de Purcellin (vendue par la Société Dragocco) | 7 % |
| PEG-6/PEG-32/Glycol Stéarate (Tefose^{R} 63 de Gattefosse) | 0,3 % |
| Sulfasalazine | 0,3 % |
| Conservateur | 0,3 % |
| Carbomer | 0,6 % |
| Crotamiton | 5 % |
| Acide glycyrrhétinique | 2 % |
| Alcool éthylique | 5 % |
| Triéthanolamine | 0,2 % |
| Eau | qsp 100 % |

### Exemple 3 : Crème pour le visage (émulsion huile dans eau)

| | |
|---|---|
| Lisophylline | 1 % |
| Stéarate de glycérol | 2 % |
| Spantide | 0,5 % |
| CGRP 8-37 | 0,25 % |
| Polysorbate 60 (Tween 60 vendu par la société ICI) | 1 % |
| Acide stéarique | 1,4 % |
| Triéthanolamine | 0,7 % |
| Carbomer | 0,4 % |
| Cyclométhicone | 8 % |
| Huile de tournesol | 12 % |
| Antioxydant | 0,05 % |
| Conservateur | 0,3 % |
| Eau | qsp 100 % |

## Revendications

1. Utilisation d'au moins un antagoniste de TNF-alpha pour la préparation d'une composition pharmaceutique ou dermatologique à application topique pour traiter les rougeurs cutanées d'origine neurogène.

2. Utilisation d'au moins un antagoniste de TNF-alpha pour la préparation d'une composition pharmaceutique ou dermatologique à application topique pour traiter la rosacée.

3. Utilisation selon la revendication 1 ou 2, caractérisée en ce que l'antagoniste de TNF-alpha est choisi parmi des molécules organiques, des molécules minérales, des extraits d'origine végétale ou animale.

4. Utilisation selon l'une quelconque des revendications précédentes, caractérisée en ce que l'antagoniste de TNF-alpha est choisi parmi la lisophylline, l'A802715, la sulfasalazine, le CDP-571 (anticorps anti-TNF-alpha), le MDL-201112.

5. Utilisation selon l'une quelconque des revendications précédentes, caractérisée en ce que l'antagoniste de TNF-alpha est utilisé en une quantité allant de 0,000001 à 10 % en poids par rapport au poids total de la composition.

6. Utilisation selon l'une quelconque des revendications précédentes, caractérisée en ce que l'antagoniste de TNF-alpha est utilisé en une quantité allant de 0,0001 à 5 % en poids par rapport au poids total de la composition.

7. Utilisation selon l'une quelconque des revendications précédentes, caractérisée en ce que la composition contient en outre au moins un agent choisi parmi les antagonistes de neuropeptide, les antagonistes de médiateur de l'inflammation, les agents antibactériens, antiparasitaires, antifongiques, anti-inflammatoires, antiseptiques, antiprurigineux, anesthésiques, antiviraux, kératolytiques, anti-radicaux libres, antiséborrhéiques, antipelliculaires, antiacnéiques et les agents modulant la différenciation et/ou la prolifération et/ou la pigmentation cutanée choisis parmi l'acide rétinoïque et ses isomères, le rétinol et ses esters, le rétinal, les rétinoïdes, la vitamine D et ses dérivés, les oestrogènes, l'acide kojique et l'hydroquinone.

8. Utilisation selon la revendication précédente, caractérisée en ce que les antagonistes de neuropeptide et les antagonistes de médiateur de l'inflammation sont choisis parmi les antagonistes de substance P, les antagonistes de CGRP, les antagonistes d'interleukine 1, les antagonistes d'histamine, les antagonistes de cyclooxygénase et les antagonistes de lipoxygénase.

9. Utilisation selon l'une quelconque des revendications précédentes, caractérisée en ce que la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles, les hydroxyacides.

10. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

11. Composition cosmétique, pharmaceutique ou dermatologique contenant, dans un milieu cosmétiquement, pharmaceutiquement ou dermatologiquement acceptable, au moins un antagoniste de TNF-alpha et au moins un antagoniste de neuropeptide et/ou un antagoniste de médiateur de l'inflammation choisi parmi les antagonistes de substance P, les antagonistes de CGRP, les antagonistes de cyclooxygénase et les antagonistes de lipoxygénase.

12. Composition selon la revendication 11, caractérisée en ce que l'antagoniste de neuropeptide ou l'antagoniste de médiateur de l'inflammation est utilisé en une quantité allant de 0,000001 à 10 % en poids par rapport au poids total de la composition.

13. Composition selon la revendication 11 ou 12, caractérisée en ce que l'antagoniste de TNF-alpha est utilisé en une quantité allant de 0,000001 à 10 % en poids par rapport au poids total de la composition.

14. Composition selon l'une quelconque des revendications 11 à 13, caractérisée en ce que l'antagoniste de TNF-alpha est utilisé en une quantité allant de 0,0001 à 5 % en poids par rapport au poids total de la composition.

15. Composition selon l'une quelconque des revendications 11 à 14, caractérisée en ce qu'elle contient en outre au moins un agent choisi parmi les agents antibactériens, antiparasitaires, antifongiques, anti-inflammatoires, antiseptiques, antiprurigineux, anesthésiques, antiviraux, kératolytiques, anti-radicaux libres, antiséborrhéiques, antipelliculaires, antiacnéiques et les agents modulant la différenciation et/ou la prolifération et/ou la pigmentation cutanée.

16. Composition selon l'une quelconque des revendications 11 à 15, caractérisée en ce que le milieu cosmétiquement, pharmaceutiquement et/ou dermatologiquement acceptable est une solution aqueuse, huileuse ou hydroalcoolique, une émulsion eau-dans-huile, une émulsion huile-dans-eau, une microémulsion, un gel aqueux, un gel anhydre, un gel huileux, un sérum, une dispersion de vésicules.

## Claims

1. Use of at least one TNFalpha antagonist for the preparation of a pharmaceutical or dermatological composition for topical application for treating red blotches on the skin of neurogenic origin.

2. Use of at least one TNFalpha antagonist for the preparation of a pharmaceutical or dermatological composition for topical application for treating rosacea.

3. Use according to Claim 1 or 2, characterized in that the TNFalpha antagonist is chosen from organic molecules, inorganic molecules and extracts of plant or animal origin.

4. Use according to any one of the preceding claims, characterized in that the TNFalpha antagonist is chosen from lisophylline, A802715, sulphasalazine, CDP-571 (anti-TNFalpha antibody) and MDL-201112.

5. Use according to any one of the preceding claims, characterized in that the TNFalpha antagonist is used in an amount ranging from 0.000001 to 10 % by weight relative to the total weight of the composition.

6. Use according to any one of the preceding claims, characterized in that the TNFalpha antagonist is used in an amount ranging from 0.0001 to 5 % by weight relative to the total weight of the composition.

7. Use according to any one of the preceding claims, characterized in that the composition further contains at least one agent chosen from neuropeptide antagonists, inflammation mediator antagonists, antibacterial, antiparasitic, antifungal, anti-inflammatory, antiseptic, antipruritic, anaesthetic, antiviral and keratolytic agents, free radical scavengers, antiseborrhoeic, antidandruff and anti-acne agents and agents that modulate differentiation and/or proliferation and/or skin pigmentation chosen from retinoic acid and its isomers, retinol and its esters, retinal, retinoids, vitamin D and its derivatives, oestrogens, kojic acid and hydroquinone.

8. Use according to the preceding claim, characterized in that the neuropeptide antagonists and the inflammation mediator antagonists are chosen from substance P antagonists, CGRP antagonists, interleukin-1 antagonists, histamine antagonists, cyclooxygenase antagonists and lipoxygenase antagonists.

9. Use according to any one of the preceding claims, characterized in that the composition contains, in addition, at least one active agent chosen from proteins or protein hydrolysates, amino acids, polyols, urea, sugars and sugar derivatives, vitamins, starch, plant extracts, essential fatty acids, ceramides, essential oils and hydroxy acids.

10. Use according to any one of the preceding claims, characterized in that the composition is an aqueous, oily or aqueous-alcoholic solution, a water-in-oil emulsion, an oil-in-water emulsion, a microemulsion, an aqueous gel, an anhydrous gel, a serum or a dispersion of vesicles, of microcapsules or of microparticles.

11. Cosmetical, pharmaceutical or dermatological composition containing, in a cosmetically, pharmaceutically or dermatologically acceptable medium, at least one TNFalpha antagonist and at least one neuropeptide antagonist and/or one inflammation mediator antagonist chosen from substance P antagonists, CGRP antagonists, cyclooxygenase antagonists and lipoxygenase antagonists.

12. Composition according to Claim 11, characterized in that the neuropeptide antagonist or the inflammation mediator antagonist is used in an amount ranging from 0.000001 to 10 % by weight relative to the total weight of the composition.

13. Composition according to Claim 11 or 12, characterized in that the TNFalpha antagonist is used in an amount ranging from 0.000001 to 10 % by weight relative to the total weight of the composition.

14. Composition according to any one of Claims 11 to 13, characterized in that the TNFalpha antagonist is used in an amount ranging from 0.0001 to 5 % by weight relative to the total weight of the composition.

15. Composition according to any one of Claims 11 to 14, characterized in that it contains, in addition, at least one agent chosen from antibacterial, antiparasitic, antifungal, anti-inflammatory, antiseptic, antipruritic, anaesthetic, antiviral and keratolytic agents, free radical scavengers, antiseborrhoeic, antidandruff and anti-acne agents and agents that modulate differentiation and/or proliferation and/or skin pigmentation.

16. Composition according to any one of Claims 11 to 15, characterized in that the cosmetically, pharmaceutically and/or dermatologically acceptable medium is an aqueous, oily or aqueous-alcoholic solution, a water-in-oil emulsion, an oil-in-water emulsion, a microemulsion, an aqueous gel, an anhydrous gel, an oily gel, a serum or a dispersion of vesicles.

## Patentansprüche

1. Verwendung mindestens eines Antagonisten von TNF-α für die Herstellung einer pharmazeutischen oder dermatologischen Zusammensetzung zur topischen Anwendung für die Behandlung von Hautrötungen neurogenen Ursprungs.

2. Verwendung mindestens eines Antagonisten von TNF-α für die Herstellung einer pharmazeutischen oder dermatologischen Zusammensetzung zur topischen Anwendung für die Behandlung der Rosacea.

3. Verwendung nach Anspruch 1 oder 2, dadurch gekennzeichnet, daß der Antagonist von TNF-α unter organischen Molekülen, anorganischen Molekülen, Extrakten pflanzlichen oder tierischen Ursprungs ausgewählt wird.

4. Verwendung nach einem der vorhergehenden Ansprüche, dadurch gekennzeichnet, daß der Antagonist von TNF-α unter Lisophyllin, A802715, Sulfasalazin, CDP-571 (Anti-TNF-α-Antikörper), MDL-201112 ausgewählt wird.

5. Verwendung nach einem der vorhergehenden Ansprüche, dadurch gekennzeichnet, daß der Antagonist von TNF-α in einer Menge von 0,000001 bis 10 Gew.-%, bezogen auf das Gesamtgewicht der Zusammensetzung, verwendet wird.

6. Verwendung nach einem der vorhergehenden Ansprüche, dadurch gekennzeichnet, daß der Antagonist von TNF-α in einer Menge von 0,0001 bis 5 Gew.-%, bezogen auf das Gesamtgewicht der Zusammensetzung, verwendet wird.

7. Verwendung nach einem der vorhergehenden Ansprüche, dadurch gekennzeichnet, daß die Zusammensetzung außerdem mindestens ein Mittel enthält, das ausgewählt wird unter den Antagonisten eines Neuropeptids, den Antagonisten eines Endzündungsmediators, antibakteriellen Mitteln, antiparasitären Mitteln, fungizid wirksamen Mitteln, entzündungshemmenden Mitteln, Antiseptika, Antipruriginosa, Anästhetika, antiviralen Mitteln, Keratolytika, Mitteln gegen freie Radikale, Antiseborrhöika, Antischuppenmitteln, Aknemitteln, Mitteln, die die Differenzierung und/oder die Proliferation und/oder die Pigmentierung der Haut modulieren, die unter Retinoesäure und deren Isomeren, Retinol und dessen Ester, Retinal, den Retinoiden, Vitamin D und dessen Derivaten, Östrogenen, Kojisäure oder Hydrochinon ausgewählt werden.

8. Verwendung nach dem vorhergehenden Anspruch, dadurch gekennzeichnet, daß die Antagonisten eines Neuropeptids und die Antagonisten eines Entzündungsmediators unter den Antagonisten der Substanz P, den Antagonisten von CGRP, den Antagonisten von Interleukin-1, den Antagonisten von Histamin, den Antagonisten von Cyclooxygenase und den Antagonisten von Lipoxygenase ausgewählt werden.

9. Verwendung nach einem der vorhergehenden Ansprüche, dadurch gekennzeichnet, daß die Zusammensetzung außerdem mindestens einen Wirkstoff enthält, der unter Proteinen und Proteinhydrolysaten, Aminosäuren, mehrwertigen Alkoholen, Harnstoff, Zuckern und Zuckerderivaten, Vitaminen, Stärke, Pflanzenextrakten, essentiellen Fettsäuren, Ceramiden, etherischen Ölen, Hydroxysäuren ausgewählt wird.

10. Verwendung nach einem der vorhergehenden Ansprüche, dadurch gekennzeichnet, daß die Zusammensetzung eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Dispersion von Vesikeln, Mikrokapseln oder Mikropartikeln ist.

11. Kosmetische, pharmazeutische oder dermatologische Zusammensetzung, die in einem kosmetisch, pharmazeutisch oder dermatologisch akzeptablen Medium mindestens einen Antagonisten von TNF-α und mindestens einen Antagonisten eines Neuropeptids und/oder Antagonisten eines Entzündungsmediators enthält, der unter den Antagonisten der Substanz P, den Antagonisten von CGRP, den Antagonisten von Cyclooxygenase und den Antagonisten von Lipoxygenase ausgewählt ist.

12. Zusammensetzung nach Anspruch 11, dadurch gekennzeichnet, daß der Antagonist eines Neuropeptids oder der Antagonist eines Entzündungsmeditors in einer Menge von 0,000001 bis 10 Gew.-%, bezogen auf das Gesamtgewicht der Zusammensetzung, verwendet wird.

13. Zusammensetzung nach Anspruch 11 oder 12, dadurch gekennzeichnet, daß der Antagonist von TNF-α in einer Menge von 0,000001 bis 10 Gew.-%, bezogen auf das Gesamtgewicht der Zusammensetzung, verwendet wird.

14. Zusamensetzung nach einem der Ansprüche 11 bis 13, dadurch gekennzeichnet, daß der Antagonist von TNF-α in einer Menge von 0,0001 bis 5 Gew.-%, bezogen auf das Gesamtgewicht der Zusammensetzung, verwendet wird.

15. Zusammensetzung nach einem der Ansprüche 11 bis 14, dadurch gekennzeichnet, daß sie außerdem mindestens ein Mittel enthält, das unter antibakteriellen Mitteln, antiparasitären Mitteln, fungizid wirksamen Mitteln, entzündungshemmenden Mitteln, Antiseptika, Antipruriginosa, Anästhetika, antiviralen Mitteln, Keratolytika, Mitteln gegen freie Radikale, Antiseborrhöika, Antischuppenmitteln, Aknemitteln und Mitteln, die die Differenzierung und/oder die Proliferation und/oder die Pigmentierung der Haut modulieren, ausgewählt ist.

16. Zusammensetzung nach einem der Ansprüche 11 bis 15, dadurch gekennzeichnet, daß das kosmetisch, pharmazeutisch und/oder dermatologisch akzeptable Medium eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein öliges Gel, ein Serum, eine Vesikeldispersion ist.
